# EUROPÄISCHE PATENTANMELDUNG

(11) **EP 2 116 824 A1**
(43) Veröffentlichungstag der Anmeldung: **11.11.2009**
(21) Anmeldenummer: 09005855.3
(22) Anmeldetag: 28.04.2009
(51) Int. Cl.: G01G 19/44, G01G 19/50, A61B 5/107

(54) **Babywaage**

(30) Priorität: 07.05.2008 DE 102008022681
(71) Anmelder: Soehnle Professional GmbH & Co. KG, 71522 Backnang (DE)
(72) Erfinder: Gerster, Stephan, 53343 Wachtberg-Pech (DE)
(74) Vertreter: Hoffmann, Jürgen

(57) **Zusammenfassung**

Eine Babywaage mit einem Gehäuse (1), einer dem Gehäuse (1) zugeordneten Waagschale (2) zur Aufnahme eines Babys, einer die Waagschale (2) tragenden Messeinrichtung zur Ermittlung des Gewichts des Babys und einer Anzeige (3) zum Anzeigen des ermittelten Gewichts ist im Hinblick auf eine komfortable Versorgung eines Babys und genauere Messergebnisse derart ausgestaltet und weitergebildet, dass die Messeinrichtung zusätzlich direkt oder indirekt eine elektrische Langenmesseinrichtung zum Vermessen des Babys und/oder ein Kopplungsmittel (14) zur Ankopplung einer elektrischen Längenmesseinrichtung zum Vermessen des Babys trägt.

## Beschreibung

Die Erfindung betrifft eine Babywaage mit einem Gehäuse, einer dem Gehäuse zugeordneten Waagschale zur Aufnahme eines Babys, einer die Waagschale tragenden Messeinrichtung zur Ermittlung des Gewichts des Babys und einer Anzeige zum Anzeigen des ermittelten Gewichts.

Babywaagen der in Rede stehenden Art sind aus der Praxis bekannt und existieren in unterschiedlichen Ausführungsformen. Eine bekannte Babywaage weist bspw. ein Gehäuse mit einer dem Gehäuse zugeordneten Waagschale zur Aufnahme eines Babys auf, wobei der Waagschale eine Messeinrichtung zur Ermittlung des Gewichts des Babys zugeordnet ist. Des Weiteren weist die Babywaage eine Anzeige zum Anzeigen des ermittelten Gewichts des Babys auf. Babywaagen der genannten Art finden ihre Anwendung sowohl im privaten Bereich als auch als geeichte Babywaage zum Wiegen von Babys in Krankenhäusern, Kinderkliniken, in Arztpraxen und bei Hebammen. Mit den bekannten Babywaagen ist eine sichere Ermittlung des Gewichts des zu wiegenden Babys möglich.

Bei der Versorgung von Babys ist jedoch nicht nur die Ermittlung des Gewichts, sondern auch die Ermittlung der Körpergröße des Babys von Bedeutung. Insbesondere unter Berücksichtigung einer Kombination des Gewichtswerts mit dem zugehörigen Längenmesswert lassen sich Rückschlüsse auf den Entwicklungs- und Ernährungszustand des Babys ziehen. Hierzu werden die Babys nach Ermittlung ihres Gewichts meist sehr umständlich mit einem separaten Längenmessstab oder einem Messband gemessen. Insbesondere in Krankenhäusern und Arztpraxen, in denen häufig eine größere Anzahl an Babys quasi gleichzeitig zu versorgen ist, besteht die Gefahr, dass das Messband oder der Messstab nicht sofort zur Hand oder gar verlegt ist. Dies behindert eine reibungslose Versorgung der Babys wesentlich.

Aus US 5,499,457 ist eine Vorrichtung zum Messen der Länge und des Gewichtes von Babys bekannt, bei der neben einer Waage eine Längenmessvorrichtung angeordnet ist. Nachteiliger Weise sind bei dieser Vorrichtung keine simultanen, hinreichend genauen Messungen von Länge und Gewicht möglich.

Auch bekannt sind Babywaagen, auf deren Wiegefläche ein Maßband aufgeklebt ist. Diese Waagen haben insbesondere den Nachteil, dass das Maßband schlecht ablesbar ist, wenn das Baby darauf oder daneben liegt. Darüber hinaus haben diese Waagen den Nachteil, dass die Messwerte nicht in elektronischer Form weiterverarbeitet oder gespeichert werden können.

Der vorliegenden Erfindung liegt daher die Aufgabe zugrunde, eine Babywaage der eingangs genannten Art anzugeben, die eine komfortable, genaue und simultane Vermessung eines Babys ermöglicht.

Erfindungsgemäß ist die voranstehende Aufgabe durch eine Babywaage gelöst, die dadurch gekennzeichnet ist, dass die Messeinrichtung zusätzlich direkt oder indirekt eine elektrische Längenmesseinrichtung zum Vermessen des Babys und/oder ein Kopplungsmittel zur Ankopplung einer elektrischen Längenmesseinrichtung zum Vermessen des Babys trägt.

Durch die Zuordenbarkeit der Längenmesseinrichtung zu dem Gehäuse oder der Waagschale ist es möglich, das zu versorgende Baby quasi gleichzeitig nicht nur zu wiegen, sondern auch bezüglich seiner Körperlänge zu messen. Mit beiden Informationen über Gewicht und Länge des Babys können somit auf einfache Weise Rückschlüsse hinsichtlich des Entwicklungs- und Versorgungszustands des Babys gezogen werden und bspw. die weitere Ernährung des Babys in geeigneter Weise geplant werden.

In erfindungsgemäßer Weise wurde erkannt, dass bei den bekannten Vorrichtungen eine genaue Gewichtsmessung unmöglich wird, wenn die Fühler oder die Messarme von mechanisch separaten Längenmessvorrichtungen mit dem zu wiegenden Baby in Kontakt stehen. Daher ist bei der erfindungsgemäßen Waage vorgesehen, dass die Messeinrichtung zur Ermittlung des Gewichts des Babys auch direkt oder indirekt die Längenmesseinrichtung trägt und stets "mitwiegt" Das "Mitwiegen" ist jedoch kein Problem, weil das bekannte Gewicht der Längenmessvorrichtung - wie beispielsweise das Gewicht der Waagschale auch - automatisch von Messergebnis abgezogen werden kann bzw. weil die Babywaage eine Tarafunktion aufweist, die die Messvorrichtung vor Beginn der Messung auf Null stellt.

Bei einer besonderen Ausführung ist vorgesehen, dass die Längenmesseinrichtung als Messschieber ausgebildet ist. Hierdurch ist nicht nur eine einfache Konstruktion, sondern auch eine einfache Handhabung der Längenmesseinrichtung durch einen Benutzer gewährleistet.

In besonders komfortabler Weise kann der Messschieber als digitaler Messschieber ausgebildet sein. Hierdurch ist eine besonders einfache und exakte Aufnahme der Längenmesswerte ermöglicht. Zur einfachen und problemlosen Anzeige der ermittelten Messwerte kann der Messschieber eine vorzugsweise digitale Anzeige aufweisen. Die ermittelten Messwerte können dabei besonders einfach auf mindestens eine Kommastelle genau abgelesen werden. Alternativ oder Zusätzlich kann auch eine analoge Anzeige aufweisen, beispielsweise um eine einfache Ablesung der ermittelten Messwerte zu ermöglichen.

In weiter konstruktiv besonders einfacher. Weise kann der Messschieber zwei ineinander schiebbare Elemente aufweisen. Im Konkreten können die Elemente teleskopartig ineinander schiebbar sein. Als einfache Elemente kommen hierbei Mehrkantrohre oder Mehrkantgestänge in Frage, bei denen aufgrund ihrer kantigen Ausbildung ein unerwünschtes Verdrehen gegeneinander sicher vermieden ist.

Auf den ineinander schiebbaren Elementen können entsprechende Längeneinheiten oder eine Längenskala ausgebildet sein. Mit einer derartigen Längenskala kann noch vor der eigentlichen Messung eine zumindest Grobabschätzung der Länge des Babys vorgenommen werden.

Hinsichtlich einer besonders genauen Längenmessung kann an den äußeren Enden der Elemente jeweils ein relativ zu dem entsprechenden Element verschwenkbares Anschlagelement angeordnet sein. Zur Messung der Länge des Babys sollte das Anschlagelement in seine Messposition gebracht werden, die im Wesentlichen eine senkrecht zu den Elementen bzw. zu dem jeweiligen Element verlaufende Position ist. Bei Nichtgebrauch der Längenmesseinrichtung und/oder im Hinblick auf eine durch die Längenmesseinrichtung unbehinderte Positionierung des Babys auf der Waagschale können die Anschlagelemente von der Babywaage bzw. der Waagschale weggeschwenkt werden, wobei die Anschlagelemente in der weggeschwenkten Position quasi eine Verlängerung der ineinander schiebbaren Elemente bilden können.

In besonders vorteilhafter Weise kann die Längenmesseinrichtung im Sinne eines Nachrüstbauteils anordenbar und wieder abnehmbar sein. Je nach Wunsch kann eine Babywaage somit zunächst auch ohne Längenmesseinrichtung erworben werden und eine Längenmesseinrichtung bei Erfordernis nachgerüstet werden. In jedem Fall kann die Babywaage für eine derartige Nachrüstung bereits vorkonfiguriert sein. Auch zu Reinigungszwecken oder zu Transportzwecken ist die Abnehmbarkeit der Längenmesseinrichtung praktisch.

Im Hinblick auf eine sichere Zuordnung der Längenmesseinrichtung zu dem Gehäuse oder zu der Waagschale kann die Längenmesseinrichtung ein weiteres Kopplungsmittel zur Anordnung an dem Gehäuse oder an der Waagschale aufweisen. Hierbei sind unterschiedliche Ausgestaltungen des Kopplungsmittels denkbar.

In weiter konstruktiv besonders einfacher Weise kann das weitere Kopplungsmittel eine vorzugsweise federvorgespannte Klammer aufweisen. Mittels einer derartigen Klammer kann die Längenmesseinrichtung einfach an dem Gehäuse oder an der Waagschale festgeklemmt werden. Eine derartige Klammer kann derart ausgestaltet sein, dass sich zwei Klammerbacken bei Betätigung der Klammer aufspreizen und diese Klammerbacken bei einem Loslassen der Klammer aufgrund der Federwirkung zusammengehen und dabei bspw. einen Rand der Waagschale ergreifen können. Mit einer derartigen Klammer ist ein besonders einfaches Anordnen der Längenmesseinrichtung am Gehäuse oder an der Waagschale ermöglicht.

Alternativ hierzu kann das weitere Kopplungsmittel in weiter konstruktiv einfacher Weise ein Einsteckelement oder eine Aufnahme aufweisen. Ein derartiges Einsteckelement oder eine derartige Aufnahme kann auf einfache Weise mit einer entsprechenden Aufnahme oder einem entsprechenden Einsteckelement des Gehäuses oder der Waagschale zusammenwirken, um einen besonders sicheren Sitz und eine besonders präzise Anordnung der Längenmesseinrichtung an dem Gehäuse oder an der Waagschale bereitzustellen.

In besonders praktischer und komfortabler Weise kann das weitere Kopplungsmittel einen elektrischen Kontakt für eine Stromversorgung der Längenmesseinrichtung über die Babywaage und/oder für eine Signalübertragung zwischen Längenmesseinrichtung und Babywaage aufweisen. Im Falle der Bereitstellung eines elektrischen Kontakts für eine Stromversorgung kann die Stromversorgung der Längenmesseinrichtung - bspw. bei einer Ausgestaltung der Längenmesseinrichtung als digitaler Messschieber - in besonders komfortabler, zuverlässiger und einfacher Weise über die Stromversorgung der Babywaage erfolgen. Die Babywaage kann entweder mittels einer Batterie oder mittels eines Netzanschlusses mit Strom versorgt werden. Eine separate Batterie oder ein separater Netzanschluss ist damit für die Längenmesseinrichtung nicht erforderlich.

Bei einer besonders exakt arbeitenden Babywaage ist vorgesehen, dass die Längenmesseinrichtung eine elektrisch von der Stromversorgung der Messeinrichtung separate, eigene Stromversorgung aufweist. Insbesondere kann vorgesehen sein, dass die Messeinrichtung direkt oder indirekt die eigene Stromversorgung mechanisch tragt. Hierdurch wird wirkungsvoll vermieden, dass ein Teil des zu messenden Gewichtes statt über die Messeinrichtung über Versorgungskabel abgeleitet wird oder dass Teile der Versorgungskabel ungewollt mitgewogen werden. Beides würde die Gewichtsmessung verfälschen. Alternativ oder Zusätzlich können vor diesem Hintergrund Mittel zum kabellosen Übertragen von Energie an die Längenmesseinrichtung vorgesehen sein. Hierbei ist es möglich, dass die Längertmessvorrichtung direkt mit der Übertragenen Energie versorgt wird oder dass die übertragene Energie zunächst, beispielsweise in einem Akku, zwischengespeichert wird.

Die Mittel zum kabellosen Übertragen arbeiten bei einer besonders effizienten Ausführungsform induktiv und/oder kapazitiv. Beispielsweise kann vorgesehen sein, dass die Mittel zum kabellosen Übertragen zumindest eine Spule aufweisen.

Bei einer Ausgestaltung eines elektrischen Kontakts für eine Signalübertragung zwischen Längenmesseinrichtung und Babywaage ist ein besonders komfortabler Betrieb der Babywaage und der Längenmesseinrichtung ermöglicht. Bspw. können mittels der Längenmesseinrichtung aufgenommene Messdaten oder ermittelte Längenmesswerte zur Babywaage hin übertragen werden und dort mittels der Anzeige der Babywaage - ggf. gemeinsam mit einem ermittelten Gewichtswert - angezeigt werden. Eine separate Anzeige der Längenmesseinrichtung wäre hierbei in kostengünstiger Weise nicht erforderlich. Alternativ oder zusätzlich hierzu wäre auch eine Signalübertragung von der Babywaage zur Längenmesseinrichtung bspw. zur Übermittlung von Steuerdaten für die Längenmesseinrichtung denkbar.

Zur Gewährleistung einer besonders sicheren Anordnung der Längenmesseinrichtung an dem Gehäuse oder an der Waagschale kann das Kopplungsmittel Rast- und/oder Verriegelungsmittel aufweisen. Bspw. kann beim Ankoppeln der Längenmesseinrichtung eine Rastnase mit dem Gehäuse oder der Waagschale eingreifen, so dass ein versehentliches Abnehmen der Längenmesseinrichtung von dem Gehäuse oder der Waagschale verhindert ist. Eine derartige Rastnase kann jedoch auf einfache Weise - durch eine bewusste Betätigung durch einen Benutzer - wieder außer Eingriff gebracht werden, so dass ein einfaches Abnehmen der Längenmesseinrichtung von dem Gehäuse oder der Waagschale möglich bleibt.

Im Hinblick auf eine einfache und sichere Anordnung der Längenmesseinrichtung am Gehäuse oder an der Waagschale kann das Gehäuse oder die Waagschale das Kopplungsmittel zur Ankopplung der Längenmesseinrichtung aufweisen. Dabei kann das Kopplungsmittel auf einfache Weise eine Aufnahme oder ein Einsteckelement aufweisen, das mit einem entsprechenden Einsteckelement oder einer entsprechenden Aufnahme der Längenmesseinrichtung zusammenwirken kann.

In besonders praktischer und komfortabler Weise kann das Kopplungsmittel einen elektrischen Kontakt für eine Stromversorgung der Längenmesseinrichtung über die Babywaage und/oder für eine Signalübertragung zwischen Längenmesseinrichtung und Babywaage aufweisen. Hinsichtlich der Vorteile und Funktionsmöglichkeiten einer Babywaage mit einer derartigen elektrischen Kontaktierung wird zur Vermeidung von Wiederholungen auf die Beschreibung der Vorteile einer derartigen elektrischen Kontaktierung im Zusammenhang mit dem oben beschriebenen Kopplungsmittel der Längenmesseinrichtung verwiesen.

Zur sicheren Anordnung der Längenmesseinrichtung zum Gehäuse oder zur Waagschale kann das Kopplungsmittel Rast- und/oder Verriegelungsmittel aufweisen.

Hierdurch ist ein unbeabsichtigtes Trennen der Längenmesseinrichtung vom Gehäuse oder von der Waagschale vermieden.

Insbesondere im Hinblick auf eine unverfälschte Gewichtsmessung ist bei einer besonderen Ausführungsform eine Signalübertragung von und/oder zur Längenmesseinrichtung kabellos realisiert. Beispielsweise kann die Signalübertragung von und/oder zur Längenmesseinrichtung über eine Funkverbindung, insbesondere eine Bluetooth-Verbindung realisiert sein. Eine derartige Signalübertragung ist in vorteilhafter Weise von irgendwelchen Kopplungsmitteln unabhängig. Letztendlich kann bei einer besonders einfachen Ausführungsform jedoch auch ein Kabel von der Längenmesseinrichtung zur Babywaage verlaufen. Hierbei können geeignete Steckmechanismen vorgesehen werden. Besonders elegant ist jedoch eine Signalübertragung über eine Funkverbindung, wodurch jegliche Verkabelung und damit das oben angesprochene Problem der Gefahr der Messwertverfälschung vermeidbar ist.

Zur Realisierung einer von der Stromversorgung der Babywaage unabhängigen Längenmesseinrichtung kann die Längenmesseinrichtung über eine Batterie betrieben sein.

Bei einer besonders kompakten Ausgestaltung der Babywaage sind das Gehäuse und die Waagschale integral ausgebildet. Letztendlich kann die Waagschale an das Gehäuse oder das Gehäuse an die Waagschale angeformt sein.

In besonders praktischer Weise können sowohl das Gewicht als auch die Länge des Babys mittels der Anzeige der Babywaage anzeigbar sein. Hierdurch ist eine besonders übersichtliche Anzeige der mittels der Babywaage ermittelten Daten und Werte ermöglicht.

Im Hinblick auf eine Weiterverarbeitung oder Archivierung der mittels der Babywaage und/oder der Längenmesseinrichtung aufgenommenen Daten und/oder Messwerte kann die Babywaage eine Einrichtung zur Ausgabe der mittels der Gewichtsmesseinrichtung und/oder der Längenmesseinrichtung aufgenommenen und/oder ermittelten Daten und/oder Messwerte aufweisen. Eine derartige Einrichtung kann je nach Anwendungsfall zur Ausgabe der Daten und/oder Messwerte über Kabel und/oder Funk ausgebildet sein. Insbesondere bei längeren Übertragungsstrecken zwischen der Babywaage und einem Verarbeitungsgerät - bspw. Ein PC - ist eine Übertragung über Funk besonders praktisch, da längere Kabelstrecken vermieden werden können.

In weiter besonders komfortabler Weise kann eine Elektronik der Babywaage und/oder der Längenmesseinrichtung derart ausgebildet sein, dass eine Umschaltung der Gewichtsmaßeinheit der Babywaage zwischen unterschiedlichen Gewichtsmaßeinheiten eine entsprechende Umschaltung der Längenmaßeinheit der Längenmesseinrichtung zwischen unterschiedlichen Längenmaßeinheiten zur Folge hat. Schaltet bspw. ein Benutzer die Gewichtsmalßeinheit der Babywaage zwischen Kilogramm und Pfund um, kann dies automatisch eine Umschaltung der Längenmaßeinheit zwischen Zentimeter und Zoll bzw. Inch bedeuten. In gleicher Weise kann eine Umschaltung der Längenmaßeinheit der Längenmesseinrichtung zu einer automatischen Umschaltung der Gewichtsmaßeinheit der Babywaage führen. Eine entsprechende Kopplung kann hierbei vorgesehen sein.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
Fig. 1 in einer schematischen und perspektivischen Ansicht ein Ausführungsbeispiel einer erfindungsgemäßen Babywaage mit einem digitalen Messschieber,
Fig. 2 in einer schematischen und perspektivischen Ansicht das Ausführungsbeispiel aus Fig. 1 von der Rückseite aus gesehen und mit abgenommener Längenmesseinrichtung, die hier ein analoger Messschieber ist,
Fig. 3 in einer schematischen und perspektivischen Ansicht das Ausführungsbeispiel aus Fig. 2 mit angekoppelter Längenmesseinrichtung und
Fig. 4 in einer schematischen und perspektivischen Ansicht zwei unterschiedliche Längenmesseinrichtungen.

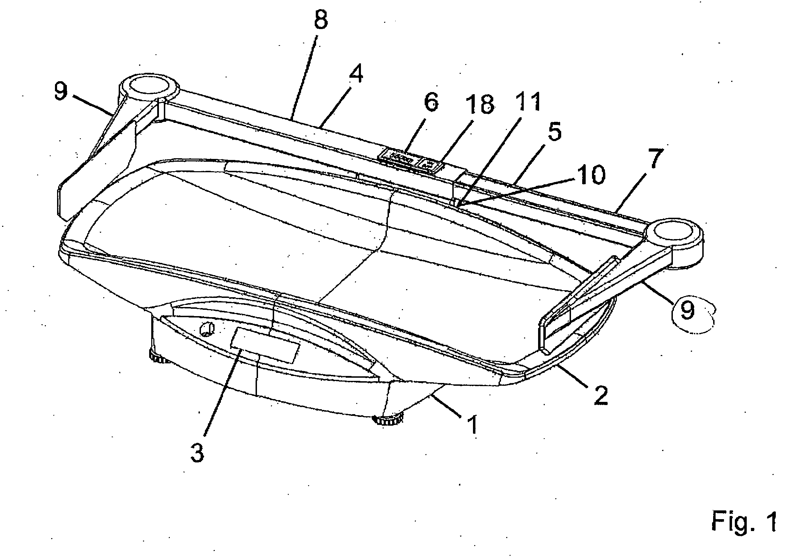

Fig. 1 zeigt in einer schematischen und perspektivischen Ansicht ein Ausführungsbeispiel einer erfindungsgemäßen Babywaage mit einem Gehäuse 1, einer dem Gehäuse 1 zugeordneten Waagschale 2 zur Aufnahme eines Babys, einer die Waagschale 2 tragenden, hier nicht gezeigten Messeinrichtung zur Ermittlung des Gewichts des Babys und einer Anzeige 3 zum Anzeigen des ermittelten Gewichts. Im Hinblick auf eine komfortable Versorgung des Babys mit konstruktiv einfachen Mitteln ist dem Gehäuse 1 oder der Waagschale 2 eine Längenmesseinrichtung 4 für das Baby zugeordnet.

Die in Fig. 1 gezeigte Babywaage steht auf einem hier nicht gezeigten Untergrund und ermöglicht sowohl die Ermittlung des Gewichts eines zu messenden Babys als auch die Feststellung seiner Körpergröße. Die angekoppelte Längenmesseinrichtung 4 ist als digitaler Messschieber 5 ausgebildet. Zum Ablesen der gemessenen Körpergröße des Babys weist der Messschieber 5 eine digitale Anzeige 6 auf. Im Konkreten weist der Messschieber 5 zwei ineinander einschiebbare Elemente 7 und 8 auf. An den äußeren Enden der Elemente 7 und 8 ist jeweils ein relativ zu dem entsprechenden Element 7 und 8 verschwenkbares Anschlagelement 9 angeordnet. Bei Nichtgebrauch der Längenmesseinrichtung 4 und/oder zum ungehinderten Positionieren des zu messenden Babys in die Waagschale 2 können die Anschlagelemente 9 von der Babywaage weg geschwenkt werden.

Die Längenmesseinrichtung 4 ist im Sinne eines Nachrüstbauteils an der Babywaage anordenbar und von dieser wieder abnehmbar. Zur sicheren Anordnung der Längenmesseinrichtung 4 an des Babywaage weist die Längenmesseinrichtung 4 ein weiteres Kopplungsmittel 10 auf. Das Kopplungsmittel 10 weist im Konkreten ein Einsteckelement 11 auf.

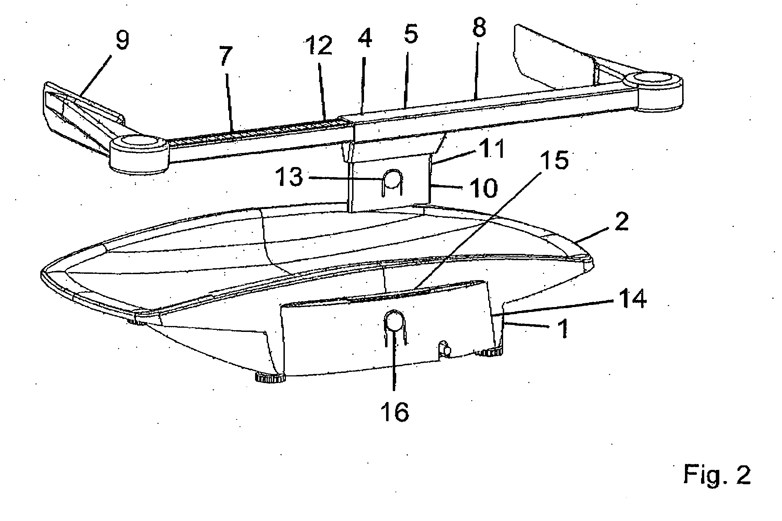

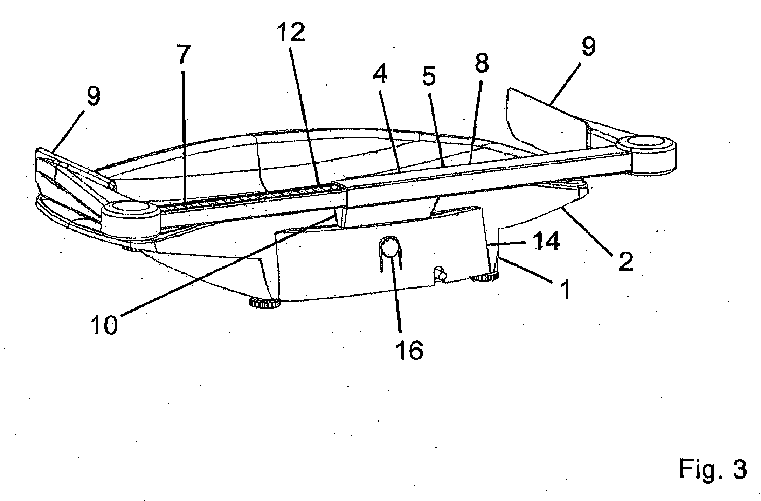

Die Fig. 2 und 3 zeigen in schematischen und perspektivischen Ansichten **-** von der Rückseite der Babywaage aus gesehen - den entkoppelten und den angekoppelten Zustand der Längenmesseinrichtung 4. Die Längenmesseinrichtung 4 ist in den Fig. 2 und 3 als analoge Längenmesseinrichtung 4 mit einer analogen Anzeige 12 ausgebildet. Die analoge Anzeige 12 weist eine Längenskala auf.

Das weitere Kopplungsmittel 10 weist am Einsteckelement 11 ein Rast- und Verriegelungsmittel 13 auf, mit dem eine Verrastung und/oder Verriegelung der Längenmesseinrichtung 4 an der Babywaage ermöglicht ist.

Hierzu weist das Gehäuse 1 ein Kopplungsmittel 14 zur Ankopplung der Längenmesseinrichtung 4 auf. Das Kopplungsmittel 14 weist eine Aufnahme 15 auf, in die das Einsteckelement 11 der Längenmesseinrichtung 4 eingesteckt werden kann, wie dies in Fig. 3 gezeigt ist. Auch das Kopplungsmittel 14 weist Rast- und/oder Verriegelungsmittel 16 auf, die mit den Rast- und/oder Verriegelungsmitteln 13 der Kopplungsmittel 11 wechselwirken.

Die Rast- und Verriegelungsmittel 16 weisen ein Federelement auf, das in eine Ausnehmung der Rast- und Verriegelungsmittel 13 der Längenmesseinrichtung 4 eingreift.

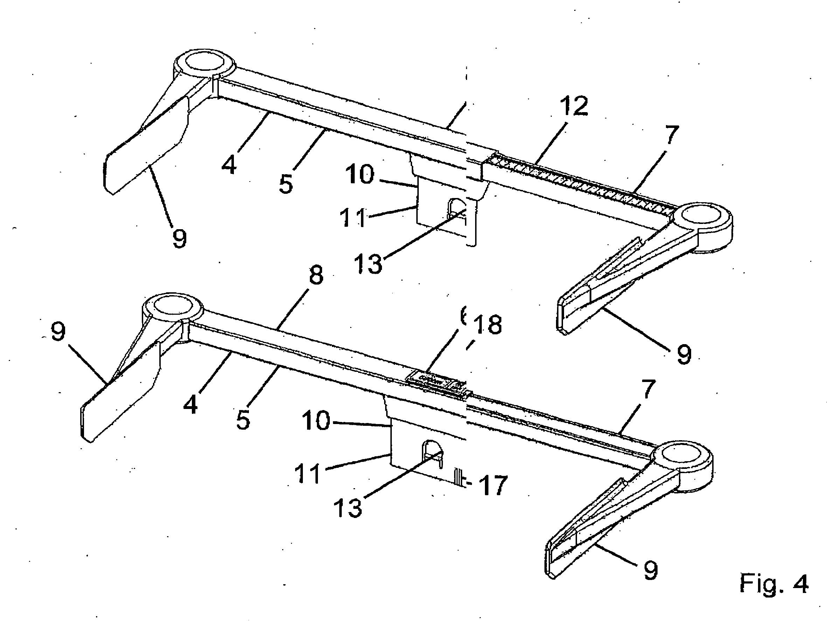

Fig. 4 zeigt in einer schematischen und perspektivischen Ansicht zwei unterschiedliche Messschieber 5, die einerseits eine analoge Anzeige 12 und andererseits eine digitale Anzeige 6 aufweisen. Die in Fig. 4 im unteren Bereich gezeigte Längenmesseinrichtung 4 weist am Einsteckelement 11 einen elektrischen Kontakt 17 für eine Stromversorgung der Längenmesseinrichtung 4 über die Stromversorgung der Babywaage auf. Eine separate Stromversorgung der digitalen Anzeige 6 über bspw. eine Batterie ist hier nicht erforderlich. Über die elektrischen Kontakte 17 kann auch eine Signalübertragung zwischen der Längenmesseinrichtung 4 und der Babywaage bzw. der Elektronik der Babywaage erfolgen. Hierdurch können mit der Längenmesseinrichtung 4 aufgenommene Messwerte oder Messdaten zur Babywaage hin übertragen oder Steuersignale von der Babywaage zur Längenmesseinrichtung 4 gesendet werden.

Zum Ein- und Ausschalten der digitalen Anzeige 6 ist ein Ein-/Aus-Schalter 18 neben der digitalen Anzeige 6 vorgesehen.

Hinsichtlich weiterer vorteilhafter Ausgestaltungen der erfindungsgemäßen Babywaage wird zur Vermeidung von Wiederholungen auf den allgemeinen Teil der Beschreibung sowie auf die beigefügten Patentansprüche verwiesen.

Schließlich sei ausdrücklich darauf hingewiesen, dass das voranstehend beschriebene Ausführungsbeispiel der erfindungsgemäßen Babywaage lediglich zur Erörterung der beanspruchten Lehre dient diese jedoch nicht auf das Ausführungsbeispiel einschränkt.

### Bezugszeichenliste

- 1: Gehäuse
- 2: Waagschale
- 3: Anzeige
- 4: Längenmesseinrichtung
- 5: Messschieber
- 6: digitale Anzeige
- 7: Element
- 8: Element
- 9: Anschlagelement
- 10: Weiteres Kopplungsmittel
- 11: Einsteckelement
- 12: analoge Anzeige
- 13: Rast- und Verriegelungsmittel
- 14: Kopplungsmittel
- 15: Aufnahme
- 16: Rast- und Verriegelungsmittel
- 17: elektrischer Kontakt
- 18: Ein-/Aus-Schalter

## Patentansprüche

1. Babywaage mit einem Gehäuse (1), einer dem Gehäuse (1) zugeordneten Waagschale (2) zur Aufnahme eines Babys, einer die Waagschale (2) tragenden Messeinrichtung zur Ermittlung des Gewichts des Babys und einer Anzeige (3) zum Anzeigen des ermittelten Gewichts, **dadurch gekennzeichnet, dass** die Messeinrichtung zusätzlich direkt oder indirekt eine elektrische Längenmesseinrichtung zum Vermessen des Babys und/oder ein Kopplungsmittel (14) zur Ankopplung einer elektrischen Längenmesseinrichtung zum Vermessen des Babys trägt.

2. Babywaage nach Anspruch 1, **dadurch gekennzeichnet, dass** die Längenmesseinrichtung (4) als Messschieber (5) ausgebildet ist oder dass die Längenmesseinrichtung (4) als digitaler Messschieber (5) ausgebildet ist.

3. Babywaage nach einem der Ansprüche 1 oder 2, **dadurch gekennzeichnet, dass** der Messschieber (5) zwei ineinander schiebbare Elemente (7, 8) aufweist, wobei an den äußeren Enden der Elemente (7, 8) jeweils ein relativ zu dem entsprechenden Element (7, 8) verschwenkbares Anschlagelement (9) angeordnet ist.

4. Babywaage nach einem der Ansprüche 1 bis 3, **dadurch gekennzeichnet, dass** die Längenmesseinrichtung (4) im Sinne eines Nachrüstbauteils anordenbar und wieder abnehmbar ist.

5. Babywaage nach einem der Ansprüche 1 bis 4, **dadurch gekennzeichnet, dass** die Längenmesseinrichtung (4) ein weiteres Kopplungsmittel (10) zur Anordnung an dem Gehäuse (1) oder an der Waagschale (2) aufweist.

6. Babywaage nach Anspruch 5, **dadurch gekennzeichnet, dass** das Kopplungsmittel (14) und/oder das weitere Kopplungsmittel (10) einen elektrischen Kontakt (17) für eine Stromversorgung der Längenmesseinrichtung (4) über die Babywaage und/oder für eine Signalübertragung zwischen Längenmesseinrichtung (4) und Babywaage aufweist.

7. Babywaage nach einem der Ansprüche 1 bis 6, **dadurch gekennzeichnet, dass** die Längenmesseinrichtung (4) eine elektrisch von der Stromversorgung der Messeinrichtung separate, eigene Stromversorgung aufweist.

8. Babywaage nach Anspruch 7, **dadurch gekennzeichnet, dass** die Messeinrichtung direkt oder indirekt die eigene Stromversorgung mechanisch trägt.

9. Babywaage nach einem der Ansprüche 1 bis 8, **dadurch gekennzeichnet, dass** Mittel zum kabellosen Übertragen von Energie an die Längenmesseinrichtung vorgesehen sind.

10. Babywaage nach Anspruch 9, **dadurch gekennzeichnet, dass** die Mittel zum kabellosen Übertragen induktiv und/oder kapazitiv arbeiten und/oder dass die Mittel zum kabellosen Übertragen zumindest eine Spule aufweisen.

11. Babywaage nach einem der Ansprüche 1 bis 10, **dadurch gekennzeichnet, dass** eine Signalübertragung von und/oder zur Längenmesseinrichtung (4) kabellos realisiert ist und/oder dass eine Signalübertragung von und/oder zur Längenmesseinrichtung (4) über eine Funkverbindung realisiert ist und/oder dass eine Signalübertragung von und/oder zur Längenmesseinrichtung (4) über eine Bluetooth-Verbindung realisiert ist.

12. Babywaage nach einem der Ansprüche 1 bis 11, **dadurch gekennzeichnet, dass** das Gehäuse (1) und die Waagschale (2) integral ausgebildet sind.

13. Babywaage nach einem der Ansprüche 1 bis 12, **dadurch gekennzeichnet, dass** sowohl das Gewicht als auch die Länge des Babys mittels der Anzeige (3) der Babywaage anzeigbar sind.

14. Babywaage nach einem der Ansprüche 1 bis 13, **dadurch gekennzeichnet, dass** die Babywaage eine Einrichtung zur Ausgabe der mittels der Gewichtsmesseinrichtung und/oder der Längenmesseinrichtung (4) aufgenommenen und/oder ermittelten Daten und/oder Messwerte aufweist.

15. Babywaage nach einem der Ansprüche 1 bis 14, **dadurch gekennzeichnet, dass** eine Elektronik der Babywaage und/oder der Längenmesseinrichtung (4) derart ausgebildet ist, dass eine Umschaltung der Gewichtsmaßeinheit der Babywaage zwischen unterschiedlichen Gewichtsmaßeinheiten eine entsprechende Umschaltung der Längenmaßeinheit der Längenmesseinrichtung (4) zwischen unterschiedlichen Längenmaßeinheiten zur Folge hat.
